(19) Europäisches Patentamt
European Patent Office
Office européen des brevets

(11) **EP 3 159 036 B1**

(12) **FASCICULE DE BREVET EUROPEEN**

(45) Date de publication et mention
de la délivrance du brevet:
**13.06.2018 Bulletin 2018/24**

(51) Int Cl.:
**A61N 1/04** *(2006.01)*      **A61N 1/05** *(2006.01)*
**A61N 1/36** *(2006.01)*

(21) Numéro de dépôt: **16194476.4**

(22) Date de dépôt: **18.10.2016**

(54) **SONDE IMPLANTABLE COMPRENANT UN MANCHON PERFORÉ**

IMPLANTIERBARE SONDE MIT EINER SIEBHÜLSE

IMPLANTABLE PROBE INCLUDING A PERFORATED SLEEVE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.10.2015 FR 1559951**

(43) Date de publication de la demande:
**26.04.2017 Bulletin 2017/17**

(73) Titulaire: **Sorin CRM SAS
92140 Clamart Cedex (FR)**

(72) Inventeur: **MEVEL, Hervé
1450 Chastre (St-Géry) (BE)**

(74) Mandataire: **Grünecker Patent- und
Rechtsanwälte
PartG mbB
Leopoldstraße 4
80802 München (DE)**

(56) Documents cités:
**US-A- 4 940 065**      **US-A- 5 251 634**
**US-A1- 2009 210 042**      **US-A1- 2013 231 726**

**Description**

L'invention concerne les dispositifs médicaux implantables actifs, plus précisément les implants permettant la stimulation d'un organe de forme cylindrique allongée, et/ou le recueil de potentiels électriques sur un tel organe.

L'invention concerne plus particulièrement la stimulation des nerfs, spécialement la stimulation du nerf vague dans le cas de thérapies dites VNS (*Vagus Nerve Stimulation*).

Toutefois, cette application ne présente aucun caractère limitatif, l'invention pouvant être utilisée pour la stimulation/détection de tout autre organe, ou encore à d'autres fins telles que la délivrance locale d'un agent actif, etc. à cet organe, dès lors que l'organe cible présente une forme cylindrique allongée.

La stimulation du système nerveux est une thérapie reconnue à l'égard de très nombreux troubles tels que l'épilepsie, la douleur, l'insuffisance cardiaque, l'apnée, l'obésité, etc.

Les dispositifs utilisés à cet effet comprennent une sonde pourvue d'une électrode implantée sur le nerf vague et un générateur délivrant des impulsions électriques sur cette électrode.

La thérapie VNS consiste à générer des salves d'impulsions répétitives, synchronisées ou non sur le rythme cardiaque selon le trouble que l'on souhaite traiter, ces impulsions se superposant aux signaux naturellement véhiculés par le système nerveux, organisé en boucle fermée. La stimulation du nerf vague pourra agir en efférence, directement vers un organe, ou en afférence, vers le cerveau pour influer sur le système nerveux central : le signal arbitraire constitué par les impulsions VNS sera alors interprété par le système nerveux central comme une sollicitation que ce dernier va tenter de compenser pour s'y opposer, et empêcher de produire ainsi les effets attendus.

L'invention concerne plus particulièrement le dispositif implanté à l'interface électrode/nerf, qui permet de maintenir les électrodes au contact du nerf ou au voisinage étroit de celui-ci.

Compte tenu de la configuration allongée et approximativement cylindrique d'un nerf périphérique tel que le nerf vague, le dispositif le plus couramment utilisé se présente sous la forme d'un manchon de forme tubulaire, enroulé autour du nerf. Le manchon est généralement réalisé en un élastomère tel que le silicone, du fait de l'excellente biocompatibilité de ce matériau, et il porte sur sa face intérieure, appliquées contre le nerf, les électrodes de stimulation (et/ou de détection).

Un manchon selon le préambule de la revendication 1 est par exemple divulgué par le US 4 602 624 A. Le manchon décrit dans ce document est réalisé à partir de deux feuilles d'élastomère laminées ensemble, l'une d'entre elles ayant été étirée au préalable dans une direction privilégiée de précontrainte. La feuille composite résultante est ensuite découpée pour donner une pièce rectangulaire qui, du fait de la précontrainte de l'une des feuilles, aura naturellement tendance, à l'état libre, à s'enrouler en spirale sur elle-même autour d'un axe perpendiculaire à la direction de précontrainte (une "spirale" étant une courbe plane s'enroulant régulièrement autour d'un point dont elle s'écarte de plus en plus).

Par rapport à un manchon rigide, le manchon décrit dans ce document présente l'avantage d'une simplicité de mise en oeuvre : il suffit au chirurgien de le dérouler, de le passer sous le nerf puis de le relâcher pour qu'il vienne de lui-même s'enrouler autour du nerf. De plus, ce manchon est auto-adaptable : en effet, immédiatement après l'implantation un processus inflammatoire normal produit un gonflement temporaire du nerf, qui disparait ensuite. Si l'on choisit un manchon spiralé souple avec un diamètre intérieur au repos légèrement inférieur au diamètre du nerf, ce manchon - avec ses électrodes - restera toujours étroitement plaqué contre le nerf même si le diamètre de celui-ci varie, et sans risque de compression excessive risquant de détériorer irréversiblement les tissus nerveux.

Ce dispositif n'est cependant pas dépourvu d'inconvénients.

Un *premier inconvénient* se présente au moment de l'implantation : pour poser le manchon, après avoir atteint le nerf cible le chirurgien tire le nerf hors de l'incision qu'il a pratiquée, pour pouvoir glisser le manchon déroulé à l'endroit choisi. Pendant cette manoeuvre, la traction exercée sur le nerf peut entrainer localement, aux extrémités du manchon, des contraintes relativement élevées sur les tissus nerveux, susceptibles d'endommager ces derniers. Une autre cause possible d'endommagement du nerf est la durée de la procédure, qui peut exposer le nerf à l'air pendant trop longtemps. Il est donc nécessaire que la procédure d'implantation du manchon soit très brève, en limitant autant que possible les manipulations du nerf. Également pendant l'implantation, les coins du manchon ont tendance à s'enrouler d'eux-mêmes et gênent l'implantation, ce qui complique la tâche du chirurgien.

Un *deuxième inconvénient,* qui apparait après l'implantation, tient au fait que le bord le plus intérieur du manchon, c'est-à-dire le bord enroulé autour du nerf, vient appuyer contre ce dernier et exerce le long de la ligne de contact une pression ayant tendance à contraindre, voire à déformer, le nerf, avec des effets potentiellement délétères. La rigidité des manchons peut aussi provoquer à leurs extrémités des forces de cisaillement inacceptables. Le risque existe aussi que lors de l'implantation le chirurgien ait laissé s'enrouler en sens inverse la partie du bord externe du manchon, qui forme alors une seconde spire en sens inverse de la première. Le rayon de courbure n'est alors plus celui qui était prévu, avec pour conséquence un risque de surépaisseur.

Un *troisième inconvénient* est lié au processus de fabrication. Comme indiqué plus haut, le manchon est réalisé en laminant ensemble deux feuilles d'élastomère, avec une précontrainte directionnelle appliquée à l'une d'entre elles. Ces feuilles étant très minces (leur épaisseur typique est d'environ 100 μm), des problèmes d'homogénéité du matériau et de tolérance de l'épaisseur peuvent apparaitre sur l'étendue de la surface d'une même feuille aussi bien qu'entre deux feuilles, ce qui limite la reproductibilité du procédé de production des manchons. Il est certes possible de pallier cet inconvénient en utilisant des feuilles de grande dimension, mais avec une incidence négative sur le processus industriel. Il est également possible d'utiliser des feuilles plus épaisses, mieux contrôlables au cours du processus, mais avec un risque accru d'endommagement du nerf du fait d'une moins grande souplesse et donc d'une moindre aptitude du manchon à se conformer à la morphologie du nerf dans la zone d'implantation.

Enfin, un *quatrième inconvénient* réside du fait de l'utilisation d'un matériel isolant comme support des électrodes et de la terminaison franche des extrémités du manchon, il en résulte la création d'électrodes virtuelles de type anode ou cathode responsables d'effets de blocage ou de stimulations non souhaitées.

Il est également connu du document US 5,251,634 un manchon pour une sonde implantable composée d'une pluralité d'hélices. Une paire de rubans conducteurs électriques est incorporée dans le matériau respectivement de deux hélices. D'autres hélices formant le manchon sont utilisées pour réaliser le maintien du manchon sur le nerf.

Cette solution technique présente également plusieurs inconvénients. Notamment, une implantation d'un tel dispositif ayant une pluralité d'hélices est difficile à mettre en place autour du nerf. De plus, les courants de stimulation à appliquer sur le nerf ne sont pas appliqués uniquement sur ce dernier car aucune protection continue sur ce manchon n'est prévue. Enfin, un manchon à hélices n'est pas adapté à recevoir des électrodes devant avoir plusieurs points de contact, notamment pour une stimulation tripolaire.

D'autres manchons pour une sonde implantable ont également été conçus notamment de forme cylindrique avec une ouverture longitudinale permettant l'implantation du manchon autour du nerf. Différents systèmes de fermeture du manchon ont alors été conçus. Toutefois, ces manchons sont de diamètre fixe et afin de ne pas abimer le nerf lors de l'implantation du manchon, celui-ci doit être choisi d'une taille supérieure au diamètre du nerf. Une bonne liaison entre le manchon et le nerf est donc impossible et par conséquent, les électrodes ne sont pas maintenues en contact avec le nerf.

Le besoin subsiste donc d'un manchon auto-enroulable en élastomère mince, pouvant être produit par un procédé industriel performant, avec un degré élevé de reproductibilité, tout en supprimant les effets secondaires connus des manchons existants.

Le but de l'invention est de résoudre ces problèmes, en proposant une nouvelle structure de manchon auto-enroulable en spirale :

- qui facilite une implantation rapide par le chirurgien, sans introduire de contraintes excessives sur le nerf, notamment qui évite de provoquer une force de cisaillement sur les bords du manchon ;
- qui respecte l'intégrité anatomique du nerf après implantation, tout en continuant d'assurer un maintien satisfaisant du manchon au site d'implantation choisi ;
- qui puisse être produit par un procédé optimisé du point de vue des contraintes industrielles et
- qui supprime la génération d'électrodes virtuelles.

À cet effet, l'invention propose une sonde implantable comprenant un manchon auto-enroulable selon la revendication 1. Selon diverses caractéristiques subsidiaires :

- les perforations sont de forme triangulaire.
- les triangles ont des angles arrondis.
- les perforations sont de forme sensiblement ronde.
- les perforations sont localisées dans au moins une zone s'étendant jusqu'à 25% de la largeur de la feuille à proximité du premier et/ou du second bord transversal, la largeur de la feuille étant définie par la distance entre le premier et le second bord transversal.
- les perforations sont formées sur le premier et/ou le second bord transversal et ledit bord est en dents de scie.
- le bord latéral extérieur est, à ses deux extrémités, raccordé aux deux bords transversaux par un bord en biseau respectif formant un angle oblique par rapport à la direction de plus grande dimension de la feuille.
- le bord en biseau s'étend sur 15 à 60 % de l'étendue de la feuille dans sa direction de plus grande dimension.
- les perforations sont localisées à proximité du premier et/ou du second bord transversal non biseauté.

Il va maintenant être décrit un exemple de mise en oeuvre de la présente invention, en référence aux dessins annexés où les mêmes références désignent d'une figure à l'autre des éléments identiques ou fonctionnellement semblables.

La Figure 1 est une vue générale de présentation d'un ensemble de stimulation VNS, montrant le générateur et le nerf vague ainsi que la sonde utilisée.
La Figure 2 est une vue d'un manchon selon l'état de la technique, enroulé autour du nerf vague pendant la procédure d'implantation.
La Figure 3 est une vue en plan du manchon, en configuration déroulée telle que ce manchon se présente au moment de sa fabrication.
La Figure 4 est une section dans le sens de l'épaisseur du manchon de la Figure 3, en vue partielle dans la région de l'un des bords, montrant notamment la structure en deux couches superposées laminées ensemble.

La Figure 5 est une vue en perspective du manchon de la Figure 3 dans sa configuration libre, enroulé sur lui-même.

La Figure 6a est une vue en plan d'un mode de réalisation du manchon selon l'invention, en configuration déroulée et les Figures 6b et 6c sont des vues partielles d'un bord longitudinal du manchon.

La Figure 7a est une vue en plan d'un deuxième mode de réalisation du manchon selon l'invention, en configuration déroulée et la Figure 7b est une vue partielle d'un bord longitudinal du manchon selon ce mode de réalisation.

La Figure 8a est une vue en plan d'un troisième mode de réalisation du manchon selon l'invention, en configuration déroulée et la Figure 8b est une vue partielle d'un bord longitudinal du manchon selon ce mode de réalisation.

La Figure 9a est une vue en plan d'un quatrième mode de réalisation du manchon selon l'invention, en configuration déroulée et la Figure 9b est une vue partielle d'un bord longitudinal du manchon selon ce mode de réalisation.

Il va maintenant être décrit un exemple de réalisation de l'invention, sous la forme d'une sonde de stimulation du nerf vague, cet exemple n'étant aucunement limitatif comme on l'a indiqué en introduction.

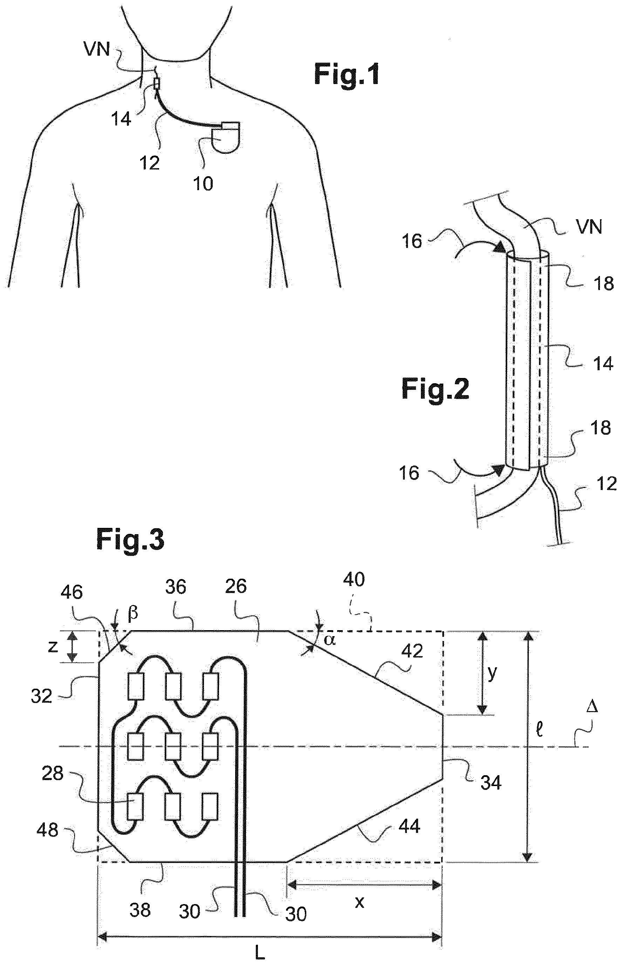

Sur la Figure 1, la référence 10 désigne le boitier d'un générateur implantable de stimulation VNS. Les impulsions de stimulation générées sont délivrées par une sonde 12 portant à sa partie distale un manchon 14 pourvu d'électrodes appliquées contre le nerf vague VN et susceptible de stimuler celui-ci par les salves d'impulsions produites par le générateur 10.

La Figure 2 est une vue d'un manchon 14 selon l'état de la technique, enroulé autour du nerf vague VN pendant la procédure d'implantation.

Pour poser le manchon, le chirurgien a dû tirer le nerf vague VN hors de l'incision qu'il avait pratiquée, pour pouvoir y glisser le manchon déroulé à l'endroit choisi. Après auto-enroulement en spirale, le manchon 14 se présente comme illustré sur la Figure 2. Dans cette configuration particulière, des contraintes sont exercées par le manchon cylindrique dans la région 16 du fait de la discontinuité de rigidité entre la partie du nerf enfermée à l'intérieur du manchon 14, et la partie libre au-delà du manchon. Cette discontinuité entre une partie où le nerf est immobilisé et celle où il est libre crée localement des contraintes à l'endroit de la transition, contraintes qui peuvent endommager les tissus nerveux.

Un autre inconvénient, également propre aux manchons de l'art antérieur, tient au fait que si l'on considère l'extrémité la plus intérieure du manchon 20 enroulé en spirale, le fait que cette extrémité soit enserrée par le reste du manchon a pour conséquence d'exercer des contraintes sur le nerf VN dans la région 22 située au voisinage de cette extrémité (contraintes schématisées par les flèches 24), qui ont pour effet de déformer le nerf de façon permanente, avec des effets potentiellement délétères.

Un autre inconvénient est lié à la création d'électrodes virtuelles tel qu'explicité ci-dessous.

La stimulation neuronale est une activation artificielle de la fibre nerveuse, l'activation étant une stimulation électrique légère, notamment par application d'une brève impulsion de courant électrique sur la membrane de la fibre nerveuse appelé le potentiel d'action.

Toutefois, l'intérieur de la membrane du nerf n'est pas accessible et le courant à travers la membrane doit être obtenu par application d'un champ de potentiel externe.

Les lois d'Ohm et de Kirchhov indiquent que le courant pénétrant la membrane (Im) est défini de la manière suivante :

$$Im \approx d(\sigma z \cdot d(Uz))/dz^2,$$

Où z est le diamètre du nerf,
σz est la conductivité électrique selon l'axe du nerf, et
Uz est le potentiel appliqué sur la membrane.

Le courant membranaire externe active la membrane par dépolarisation alors que le courant membranaire interne inhibe l'activation ou la propagation des potentiels d'action par hyperpolarisation.

Le matériau de support des électrodes, en général un élastomère de silicone, présente une très faible conductivité comparé aux fluides et aux tissus biologiques entourant le manchon.

Ainsi, le courant généré par les électrodes le long du nerf va rencontrer un changement brusque de conductivité du matériau (σz) aux deux extrémités du manchon. Il en résulte une génération de courants internes correspondant à une électrode virtuelle de type anode, ou de courants externes correspondant à une électrode virtuelle de type cathode, à travers la membrane. Ces électrodes virtuelles peuvent stimuler le nerf ou au contraire bloquer la transmission d'un potentiel d'action. Ces effets sont dans la plupart des cas une nuisance puisqu'ils ne peuvent être contrôlés.

Un autre inconvénient à ce dispositif de l'état de la technique est dû au fait que les anodes virtuelles et cathodes virtuelles n'agissent pas uniquement sur le nerf mais également sur l'environnement du manchon entourant le nerf, à savoir les muscles, la peau ou d'autres nerfs provoquant pour le patient des effets indésirables.

Enfin, un tel manchon ayant une certaine rigidité, notamment sur les bords du manchon, peut endommager le nerf par la création d'une pression sur le nerf, tendant à contraindre et déformer le nerf. En outre, les bords du manchon peuvent exercer des forces de cisaillement sur le nerf.

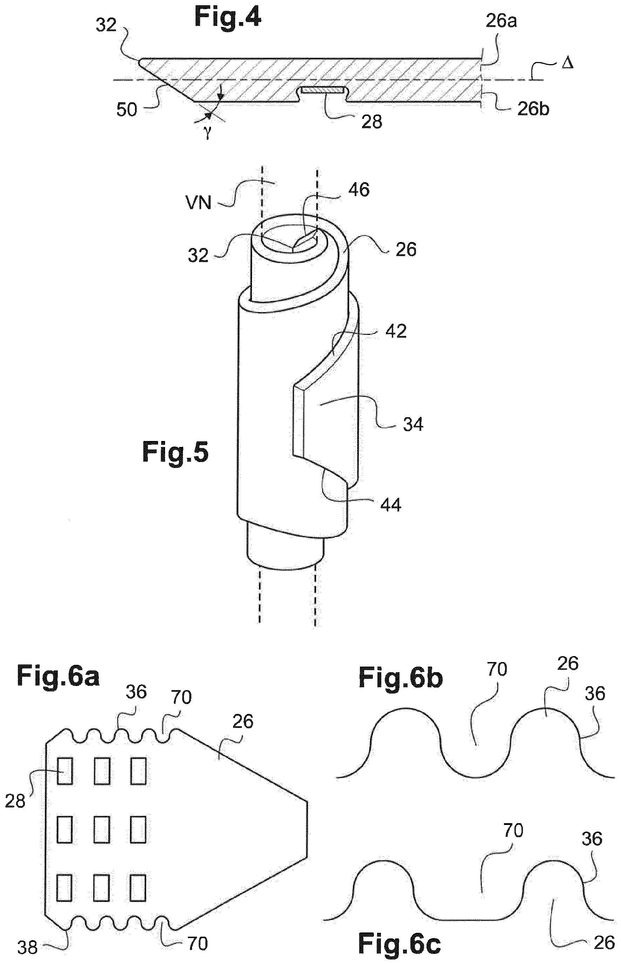

Ces différents inconvénients, ainsi que ceux exposés en introduction, peuvent être résolus par un manchon réalisé selon les enseignements de l'invention, illustré aux Figures 3 à 9, notamment aux Figures 6 à 9.

La Figure 3 est une vue en plan du manchon selon l'invention, en configuration déroulée telle que ce manchon se présente au moment de sa fabrication.

Le manchon 26 selon l'invention est réalisé à partir de deux feuilles d'élastomère 26a et 26b (Figure 4) laminées ensemble, par exemple de silicone, l'une des feuilles ayant été soumise au préalable à une précontrainte d'étirement dans la direction Δ, qui est dans cet exemple la direction de plus grande dimension de la feuille 26. Comme expliqué dans le US 4 602 624 A précité, cette technique permet de rendre le manchon auto-enroulable en spirale lorsque la feuille 26, après fabrication du manchon, ne sera plus soumise à aucune contrainte extérieure, conduisant à la configuration enroulée illustré Figure 5. De la sorte, la feuille 26 est précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe, avec la première face portant les électrodes tournée vers l'intérieur.

Le silicone est choisi préférentiellement comme matériau de base pour le manchon implantable en raison de ses excellentes propriétés de biocompatibilité, tant au niveau de la biotolérance (l'implant n'induit pas de dommage à l'hôte : absence de toxicité et d'endommagement mécanique des tissus) que de sa biostabilité (l'implant résiste aux conditions induites par l'hôte).

La feuille 26 porte, dans la région destinée à venir en contact avec le nerf vague après enroulement (la région située à gauche sur la Figure 3) un certain nombre d'électrodes 28 rapportées en surface de la feuille ou enfoncées dans l'épaisseur du matériau élastomère. Ces électrodes 28 sont reliées à des fils 30 destinés à être reliés au générateur d'impulsions 10. Dans l'exemple illustré sur la Figure 3, ces électrodes 28 sont distribuées de manière uniforme le long de l'axe d'enroulement du manchon 26 et elles sont reliées entre elles de manière à constituer une matrice de contacts de type quasi-tripôle (anode/cathode/anode ou l'inverse) reliés aux microcâbles 30 correspondants.

Le manchon est réalisé à partir de la feuille 26 qui présente une forme rectangulaire, avec un bord latéral intérieur 32 formant une première largeur (qui viendra à l'intérieur de la spirale après enroulement du manchon), un deuxième bord latéral extérieur 34 opposé formant une seconde largeur (qui viendra à l'extérieur du manchon après enroulement), et un premier bord transversal 36 réunissant des premières extrémités homologues du premier bord latéral 32 et du second bord latéral 34, et un second bord transversal 38 opposé réunissant des secondes extrémités homologues du premier bord latéral 32 et du second bord latéral 34.

Les coins à angle droit de cette feuille rectangulaire sont découpés (par estampage, découpe à la lame ou tout autre processus industriel adapté) de manière à éliminer les régions délimitées par les tirets 40, formant ainsi des bords en biseau 42, 44, 46, 48. Dans la région située du côté 34, les bords en biseau 42 et 44 forment avec les longueurs 36, 38 (elles-mêmes parallèles à l'axe Δ) un angle $\alpha$ de 20° à 45° par exemple, de sorte que l'étendue de la partie 40 éliminée pour former le biseau s'étend sur une longueur $x$ de l'ordre de 15 à 60 % de la longueur totale $L$ de la feuille 26, et sur une largeur $y$ de l'ordre de 20 à 50 % de la largeur totale $\ell$ de la feuille 26. Quant aux biseaux 46, 48 du côté opposé, ceux-ci forment un angle $\beta$ de 30° à 60° et s'étendent sur une largeur $z$ de 10 à 25 % de la largeur totale $\ell$ de la feuille 26.

Par ailleurs, dans le sens de l'épaisseur, l'extrémité 32 forme un bord chanfreiné 50, le chanfrein étant tourné vers la face de la feuille qui sera appliquée contre le nerf (c'est-à-dire la face portant les électrodes 28). Ce chanfrein est incliné d'un angle $\gamma$ compris entre 20° et 45° par exemple.

Avec la configuration précédemment décrite, dans sa configuration enroulée le manchon selon l'invention prend la forme illustrée en Figure 5, avec un aspect ressemblant à un croissant de viennoiserie (de forme droite), du fait des bords en biseau 42, 44, qui donnent à la partie enroulée côté extérieur une forme de languette.

Cette languette facilite les opérations d'implantation, dans la mesure où le manchon peut être manipulé sans risque de replier des coins du manchon vers l'intérieur, ce qui formerait des surépaisseurs dommageables.

De plus, la forme en "croissant" rend immédiatement visible un enroulement inversé par mégarde, c'est-à-dire où la région portant les électrodes (à gauche sur la Figure 3) viendrait recouvrir la région opposée (la région droite sur la Figure 3), au lieu que ce soit l'inverse. Comme les biseaux 46, 48 sont de beaucoup plus petite dimension que les biseaux 42, 44, la forme typique avec languette comme sur la Figure 5 serait absente, révélant immédiatement l'enroulement à l'envers.

Un autre avantage de cette forme en croissant réside dans le gradient de rigidité du manchon en configuration enroulée, la rigidité diminuant progressivement depuis le centre du manchon jusqu'aux extrémités. La plus grande souplesse aux extrémités permet d'exercer localement moins de contraintes sur le nerf (à la différence des manchons de la technique antérieure, comme illustré Figure 2), tandis que dans la région centrale le serrage exercé par le manchon est maximal, ce qui permet de bien maintenir les électrodes en appui contre le nerf.

Pour réaliser le manchon, il est possible d'utiliser des feuilles d'épaisseur relativement mince (de l'ordre de 100 μm), ce qui conduit à des manchons très souples, et donc très bien tolérés, sans compromis sur la facilité de pose et avec une transition très progressive entre le nerf et le manchon.

Un manchon conformément à l'invention pour une sonde implantable, apte à être enroulé autour d'un organe de forme cylindrique allongée tel qu'un nerf (VN), est montré aux Figures 6 à 9 afin d'illustrer des exemples de réalisation de l'invention.

Tel qu'illustré sur les Figures 6 à 9 et conformément à l'invention, la feuille 26 comprend une pluralité de perforations 70, localisées à proximité du premier et / ou du second bord transversal 36, 38.

Les perforations 70 situées à proximités du / des bords transversaux permettent, une fois le manchon en place autour du nerf, d'éviter la création d'électrodes virtuelles.

En effet, ces perforations vont d'une part réduire l'épaisseur du manchon aux extrémités de celui-ci et d'autre part réduire la différence de conductivité aux extrémités du manchon, ce qui a pour conséquence de supprimer la génération non souhaitée d'électrodes virtuelles.

On entend par perforation, l'enlèvement de matière, de sorte à former un trou traversant ou borgne, ou bien une indentation (perforation ouverte latéralement débouchant du côté du bord transversal) sur le manchon à proximité du / des bords transversaux, c'est-à-dire sur le / les bords transversaux ou dans une zone s'étendant jusqu'à 25% de la largeur de la feuille au niveau de chaque bord transversal, la largeur de la feuille étant définie par la distance entre le premier et le second bord transversal. Cet enlèvement de matière peut être réalisé selon une multitude de formes, par exemple ronde, triangulaire, ovale, en dent de scie, etc.

En outre, les perforations à proximité du ou des bords transversaux peuvent être présentes sur tout ou partie de la longueur des bords transversaux.

De manière préférentielle, les perforations à proximité du ou des bords transversaux sont réalisées sur tout ou partie de la longueur des bords transversaux non biseautés.

Des exemples de mode de réalisation sont illustrés sur les Figures 6 à 9, toutefois, d'autres formes de perforations peuvent être réalisées.

En Figures 6a, 6b et 6c, il est illustré une feuille 26 comprenant des perforations sur les deux bords transversaux 36, 38. Toutefois, selon une alternative de réalisation, non illustrée, les perforations sont formées sur le premier ou le second bord transversal 36, 38.

Selon ce mode de réalisation, les perforations créées sur les bords transversaux 36, 38 forment des indentations des bords en dents de scie.

Les dents de scie sont arrondies par exemple de manière à former un ou des bords transversaux ondulés tel que montré en Figure 6b.

La hauteur de l'ondulation est par exemple comprise entre 1 et 2 millimètres.

L'ondulation peut être réalisée sur toute la longueur du ou des bords transversaux ou au contraire est présente seulement sur une partie de la longueur du ou des bords transversaux, par exemple sur une partie de la longueur du ou des bords transversaux non biseautés.

En outre, l'ondulation peut être réalisée de manière régulière ou non.

Enfin, le pas d'ondulation peut être proche tel qu'illustré en Figure 6b ou au contraire être éloigné tel que montré en Figure 6c.

Les modes de réalisation illustrés aux Figures 6b et 6c présentent également l'avantage d'avoir des formes courbes sur les bords transversaux évitant de la sorte l'ancrage de tissu fibrotique.

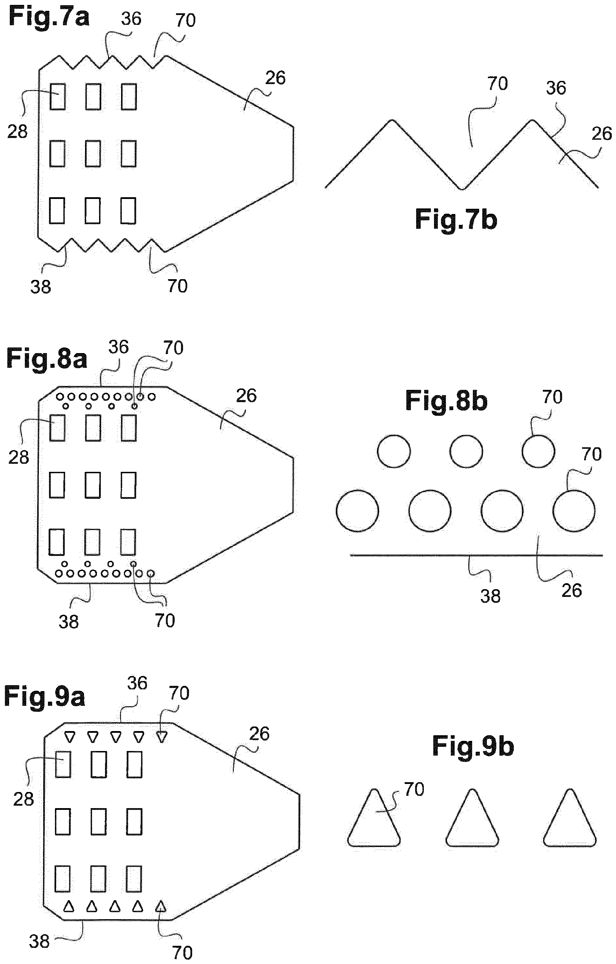

En Figures 7a et 7b, il est illustré une feuille 26 comprenant des perforations 70 sur les bords transversaux 36, 38 de manière à former des indentations sur des bords transversaux en forme de dents de scie non arrondies. Autrement dit, les dents de scie illustrées en Figure 7b sont en forme de triangle. Afin de limiter le développement de tissu fibrotique, le sommet des dents forme un angle obtus.

Selon un exemple de réalisation, la distance entre deux sommets de dents est comprise entre 2 et 4 millimètres. En outre, la hauteur de chacune des dents est comprise par exemple entre 1 et 2 millimètres.

Selon un exemple particulier de réalisation, les dents formées sur les bords transversaux ont toutes la même hauteur. Toutefois, selon une alternative de réalisation, les dents formées sur les bords transversaux sont de hauteurs différentes.

En outre, selon une première alternative de réalisation des bords transversaux à dents de scie, les dents sont jointes les unes aux autres de manière contiguë.

Selon une seconde alternative de réalisation de ces bords transversaux à dents de scie, les dents sont espacées les unes des autres tel qu'illustré en Figure 6c.

Selon un mode de réalisation particulier, les bords transversaux en dents de scie peuvent être formés alternativement ou non, de dents arrondies et de dents en forme de triangle.

Selon un autre mode de réalisation illustré aux Figures 8 et 9, les perforations sont localisées dans au moins une zone s'étendant jusqu'à 25% de la largeur de la feuille au niveau d'un ou des bords transversaux 36, 38, la largeur de la feuille étant définie par la distance entre le premier et le second bord transversal. Ces perforations peuvent s'étendre sur tout ou partie de la longueur du ou des bords transversaux, notamment sur tout ou partie de la longueur du ou des bords transversaux non biseautés.

Selon ce mode de réalisation, les perforations sont réalisées dans une ou des zones voisines aux bords transversaux réduisant de la sorte l'épaisseur du manchon au niveau des extrémités du manchon lorsque celui-ci est enroulé. Ainsi, conformément à l'invention, on réduit la différence de conductivité aux extrémités du manchon. Par conséquent, la génération non souhaitée d'électrodes virtuelles est supprimée.

En particulier, il est montré en Figure 8a des perforations 70 réalisées à proximité des deux bords transversaux non biseautés 36 et 38. Bien entendu, des perforations peuvent être réalisées uniquement sur un bord transversal 36 ou 38.

Selon le mode de réalisation illustré en Figure 8a, les perforations 70 sont de forme sensiblement ronde, ces perforations pouvant être traversantes ou borgnes, par exemple réalisées sur une seule feuille d'élastomère 26a ou 26b. Ces perforations peuvent également être de forme par exemple sensiblement oblongue. Du fait de l'absence d'angle dans ces formes de perforations, il ne peut y avoir un ancrage de tissu fibrotique.

Les perforations de forme sensiblement ronde ou oblongue, tel qu'illustré en Figure 8b sont situées à proximité d'un bord transversal, notamment du bord transversal non biseauté, sur une ou plusieurs rangées, par exemple sur deux rangées. Selon un exemple de réalisation, les perforations de forme sensiblement ronde sont positionnées en quinconce.

Selon un exemple de réalisation, les performations sont de diamètre compris entre 10 micromètres et 500 micromètres.

La Figure 8b illustre deux rangées de perforations 70 dont le diamètre des perforations de la première rangée, rangée la plus proche du bord transversal, est différent, notamment supérieur, au diamètre des perforations de la seconde rangée. Alternativement, le diamètre des perforations de la première rangée, à proximité du bord transversal, peut être inférieur au diamètre des perforations de la seconde rangée. Selon un mode de réalisation préféré, il est avantageux d'avoir un gradient de perforations en direction du ou des bords transversaux.

En Figure 9a, il est représenté un autre exemple de réalisation, dans lequel des perforations sont réalisées sous forme de triangle, localisées dans au moins une zone s'étendant jusqu'à 25% de la largeur de la feuille au niveau d'un ou des bords transversaux 36, 38, la largeur de la feuille étant définie par la distance entre le premier et le second bord transversal.

Les triangles, selon un mode de réalisation particulier, ont des angles arrondis afin d'éviter un ancrage des tissus fibrotiques. Les côtés des triangles sont de longueur par exemple comprise entre 200 micromètres et 1 millimètre.

La Figure 9a illustre la présence d'une rangé de perforations en forme de triangle de chaque côté des bords transversaux. Toutefois, il peut être prévu d'avoir plusieurs rangés de perforations de forme triangulaire. De même, des perforations triangulaires de plusieurs dimensions peuvent aussi être mises en oeuvre.

Bien entendu, les modes de réalisation illustrés aux Figures 6a à 9a peuvent être combinés.

## Revendications

1. Sonde implantable comprenant un manchon apte à être enroulé autour d'un organe de forme cylindrique allongée tel qu'un nerf (VN), ce manchon comprenant une feuille (26) en matériau élastiquement déformable portant au moins une électrode (28) de détection/stimulation sur une première face de la feuille,
la feuille (26) étant précontrainte de manière à permettre son auto-enroulement depuis une position initiale où la feuille est maintenue sous contrainte à l'état déployé jusqu'à une position finale où la feuille est librement enroulée en spirale en formant un manchon autour de l'organe, avec la première face portant les électrodes tournée vers l'intérieur,
la feuille (26) étant délimitée par un premier bord latéral (34) formant un bord latéral extérieur du manchon, un second bord latéral (32) formant un bord latéral intérieur du manchon , et un premier bord transversal (36) réunissant des premières extrémités homologues du premier bord latéral (34) et du second bord latéral (32), et un second bord transversal (38) opposé réunissant des secondes extrémités homologues du premier bord latéral (34) et du second bord latéral (32),
**caractérisée en ce que** la feuille (26) comprend une pluralité de perforations (70) localisées à proximité du premier et/ou du second bord transversal (36, 38).

2. Sonde implantable selon la revendication 1, **caractérisée en ce que** les perforations sont de forme triangulaire.

3. Sonde implantable selon la revendication 2, **caractérisée en ce que** les triangles ont des angles arrondis.

4. Sonde implantable selon la revendication 1, **caractérisée en ce que** les perforations sont de forme sensiblement ronde.

5. Sonde implantable selon l'une des revendications précédentes, **caractérisée en ce que** les perforations sont localisées dans au moins une zone s'étendant jusqu'à 25% de la largeur de la feuille à proximité du premier et/ou du second bord transversal, la largeur de la feuille étant définie par la distance entre le premier et le second bord transversal.

6. Sonde implantable selon l'une des revendications 1 à 4, **caractérisée en ce que** les perforations sont formées sur le premier et/ou le second bord transversal et ledit bord est en dents de scie.

7. Sonde implantable selon l'une quelconque des revendications précédentes, **caractérisée en ce que** le bord latéral extérieur (34) est, à ses deux extrémités, raccordé aux deux bords transversaux (36, 38) par un bord en biseau respectif formant un angle oblique ($\alpha$) par rapport à la direction ($\Delta$) de plus grande dimension de la feuille.

**8.** Sonde implantable selon la revendication 7, dans laquelle le bord en biseau (42, 44) s'étend sur 15 à 60 % de l'étendue (L) de la feuille dans sa direction (Δ) de plus grande dimension.

**9.** Sonde implantable selon l'une quelconque des revendications 7 ou 8, **caractérisée en ce que** les perforations sont localisées à proximité du premier et/ou du second bord transversal non biseauté.

**Patentansprüche**

**1.** Implantierbare Sonde, umfassend eine Hülse, die um ein zylindrisches längliches Organ, wie ein Nerv (VN) gewickelt werden kann, wobei diese Hülse ein Blatt (26) aus einem elastisch verformbaren Material umfasst, das mindestens eine Sensor- und Stimulationselektrode (28) auf einer ersten Seite des Blatts trägt,
wobei das Blatt (26) derart vorgespannt ist, dass es sich eigenständig aus einer Anfangsstellung, in der das Blatt zwangsweise ausgerollt ist, in eine Endstellung, in der das Blatt spiralförmig frei aufgerollt ist, aufrollen kann und eine Hülse um das Organ gebildet wird, wobei die erste Seite die nach innen gewandten Elektroden trägt,
wobei das Blatt (26) durch eine erste Seitenkante (34), die eine äußere Seitenkante der Hülse bildet, eine zweite Seitenkante (32), die eine innere Seitenkante der Hülse bildet, und eine erste Querkante (36), die erste entsprechende Enden der ersten Seitenkante (34) und der zweiten Seitenkante (32) verbindet, und eine zweite gegenüberliegende Querkante (38), die zweite entsprechende Enden der ersten Seitenkante (34) und der zweiten Seitenkante verbindet, begrenzt ist,
**dadurch gekennzeichnet, dass** das Blatt (26) eine Vielzahl von Perforationen (70) umfasst, die sich in der Nähe der ersten und/oder der zweiten Querkante (36, 38) befinden.

**2.** Implantierbare Sonde nach Anspruch 1, **dadurch gekennzeichnet, dass** die Perforationen dreieckig sind.

**3.** Implantierbare Sonde nach Anspruch 2, **dadurch gekennzeichnet, dass** die Dreiecke gerundete Ecken aufweisen.

**4.** Implantierbare Sonde nach Anspruch 1, **dadurch gekennzeichnet, dass** die Perforationen eine im Wesentlichen runde Form aufweisen.

**5.** Implantierbare Sonde nach einem der vorhergehenden Ansprüche, **dadurch gekennzeichnet, dass** sich die Perforationen in der Nähe der ersten und/oder zweiten Seitenkante, in mindestens einem Gebiet befinden, der sich bis zu 25% der Breite des Blatts erstreckt, wobei die Breite des Blatts dem Abstand zwischen der ersten und der zweiten Querkante entspricht.

**6.** Implantierbare Sonde nach einem der Ansprüche 1 bis 4, **dadurch gekennzeichnet, dass** die Perforationen auf der ersten und/oder zweiten Seitenkante gebildet sind und die Kante sägezahnförmig ist.

**7.** Implantierbare Sonde nach einem der vorhergehenden Ansprüche, **dadurch gekennzeichnet, dass** die äußere Seitenkante (34) an ihren beiden Enden mit den zwei Querkanten (36, 38) durch eine jeweilige schräge Kante verbunden ist, die einen schrägen Winkel (α) zur Richtung (Δ) der größten Abmessung des Blatts bildet.

**8.** Implantierbare Sonde nach Anspruch 7, in der sich die schräge Kante (42, 44) über 15 bis 60% der Ausdehnung (L) des Blatts in Richtung (Δ) seiner größten Abmessung erstreckt.

**9.** Implantierbare Sonde nach einem der Ansprüche 7 bis 8, **dadurch gekennzeichnet, dass** sich die Perforationen in der Nähe der ersten und/oder zweiten nicht schrägen Kante befinden.

**Claims**

**1.** An implantable probe comprising a sleeve that is adapted to be wound around an elongate organ of cylindrical shape, such as a nerve (VN), the sleeve comprising a sheet (26) of elastically deformable material carrying at least one detection/stimulation electrode (28) on a first face of the sheet,
wherein the sheet (26) is prestressed so as to allow it to wind automatically from an initial position, in which the sheet is kept stressed in the deployed state, to a final position, in which the sheet is wound freely in a spiral to form a sleeve around the organ, with the first face of the sheet, which carries the electrodes, being directed towards the inside,
wherein the sheet (26) is delimited by a first lateral edge (34), which forms an outer lateral edge of the sleeve, a second lateral edge (32), which forms an inner lateral edge of the sleeve, and a first transverse edge (36), which joins first counterpart ends of the first lateral edge (34) and of the second lateral edge (32), and an opposite second transverse edge (38), which joins second counterpart ends of the first lateral edge (34) and the second lateral edge (32).
**characterized in that** the sheet (26) comprises a plurality of perforations (70) located close to the first and/or second transverse edge (36, 38).

**2.** The implantable probe according to claim 1, **char-**

**acterized in that** the perforations have a triangular shape.

3. The implantable probe according to claim 2, **characterized in that** the triangles have rounded angles.

4. The implantable probe according to claim 1, **characterized in that** the perforations have a substantially round shape.

5. The implantable probe according to one of the aforementioned claims, **characterized in that** the perforations are located at least in an area extending across 25% of the width of the sheet close to the first and/or second transverse edge, the width of the sheet being defined as the distance between the first and the second transverse edge.

6. The implantable probe according to one of the claims 1 to 4, **characterized in that** the perforations are formed on the first and/or second transverse edge and said edge has a sawtooth-like shape.

7. The implantable probe according to one of the aforementioned claims, **characterized in that** the outer lateral edge (34) is joined at both ends with the two transverse edges (36, 38) by a respective beveled edge forming an oblique angle ($\alpha$) with respect to the direction ($\Delta$) of the biggest measurement of the sheet.

8. The implantable probe according to claim 7, in which the beveled edge (42, 44) extends across 15 to 60% of the extent ($L$) of the sheet in its direction ($\Delta$) of biggest measurement.

9. The implantable probe according to one of the claims 7 or 8, **characterized in that** the perforations are located close to the first and/or second non beveled transverse edge.

# Fig.1

VN

14

12

10

# Fig.2

16

VN

18

14

18

16

12

# Fig.3

46  β  36  26  40

z

32

28

48

38  30  30

L

42

y

α

ℓ  Δ

34

44

x

# Fig.4

# Fig.5

# Fig.6a

# Fig.6b

# Fig.6c

Fig.7a

Fig.7b

Fig.8a

Fig.8b

Fig.9a

Fig.9b

**EP 3 159 036 B1**

## RÉFÉRENCES CITÉES DANS LA DESCRIPTION

**Documents brevets cités dans la description**

- US 4602624 A

- US 5251634 A